# EUROPÄISCHE PATENTSCHRIFT

(11) **EP 0 953 558 B1**
(45) Veröffentlichungstag und Bekanntmachung des Hinweises auf die Patenterteilung: **21.11.2001**
(21) Anmeldenummer: 99105582.3
(22) Anmeldetag: 13.03.1999
(51) Int. Cl.: C07C 37/60

(54) **Verfahren zur Herstellung von Hydroxylgruppen enthaltenden aromatischen Verbindungen**
Process for the preparation of aromatic compounds containing hydroxyl groups
Procédé pour la préparation de composés aromatiques contenant des groupes hydroxyles

(30) Priorität: 30.04.1998 DE 19819194; 01.10.1998 DE 19845136; 18.12.1998 DE 19858505
(43) Veröffentlichungstag der Anmeldung: 03.11.1999
(73) Patentinhaber: CREAVIS Gesellschaft für Technologie und Innovation mbH, 45764 Marl (DE)
(72) Erfinder: Kühnle, Adolf Dr., 45770 Marl (DE); Duda, Mark Dr., 45770 Marl (DE)
(74) Vertreter: Olbricht, Gerhard Dr.

(56) Entgegenhaltungen:
- US-A- 5 055 623
- US-A- 5 811 599

## Beschreibung

Die Erfindung betrifft die einstufige Herstellung eines eine Hydroxylgruppe enthaltenden Aromaten durch selektive, katalytische Oxidation eines aromatischen Kohlenwasserstoffs.

Um zu Hydroxylgruppen enthaltenden aromatischen Verbindungen wie z.B. zu Phenol zu gelangen war es bislang nicht möglich, Benzole direkt durch selektive Oxidation in einer Stufe und in hohen Ausbeuten in entsprechende Hydroxyverbindungen zu überführen. Entweder wurde der aromatische Ring überhaupt nicht durch das Oxidationsmittel angegriffen oder er wurde durch dieses zerstört. Es bildeten sich hauptsächlich Kohlendioxid und Koks.

Unter wirtschaftlichen Aspekten war es bislang nur über mehrere Zwischenstufen möglich, eine Hydroxylgruppe in das aromatische System einzuführen.

Technisch durchgesetzt für die Herstellung von Phenolen ausgehend von Benzolen hat sich das Cumolverfahren. Hierbei wird üblicherweise das aus Benzol und Propen hergestellte Cumol peroxidiert und danach das Oxidationsprodukt in Phenol und Aceton gespalten.

Weiterhin ist ausgehend von Toluol der Benzoesäureprozeß im Einsatz, wobei die aus Toluol hergestellte Benzoesäure zu Phenol decarboxyliert werden kann. Der Decarboxylierschritt, d.h. der Verlust eines organisch gebundenen Kohlenstoffs, zehrt jedoch einen etwaigen Preisvorteil von Toluol gegenüber Benzol bereits in dieser Stufe auf. Der Prozeß ist daher nur interessant, wenn das Zielprodukt Benzoesäure ist und freie Kapazitäten für die Herstellung von Phenol genutzt werden.

Andere Verfahren zur Herstellung von Phenol z.B. über Chlorbenzol (Chlorierung bzw. Oxychlorierung von Benzol) oder das Sulfonierungsverfahren (Herstellung von Benzolsulfonsäure) haben sich als unwirtschaftlich erwiesen. Gründe waren z.T. die unbefriedigende Selektivität, Korrosionsprobleme sowie der Anfall unerwünschter Nebenprodukte.

Auch das Cyclohexanolverfahren (Hydratisierung von Cyclohexen in der 1. Stufe) ist nicht wirtschaftlich. Das Verfahren verläuft über zu viele Stufen, um zum Zielprodukt Phenol zu gelangen.

Aus diesem Grunde wird weltweit der größte Anteil an Phenol über die oben erwähnte Cumolroute hergestellt. Da hierbei jedoch auch Aceton anfällt, ist die Wirtschaftlichkeit dieses Verfahren von den Marktpreisen für Phenol und Aceton abhängig.

Um die Abhängigkeit von dem Koppelprodukt Aceton zu umgehen, konzentrieren sich viele Versuche auf die selektive Oxidation von Benzol bzw. Benzolderivaten. So beschreiben beispielsweise die US-Patente Nr. 5.055.623, Nr. 5.672.777 und Nr. 5.110.995 die Oxidation von Benzol mit Distickstoffmonoxid an geeigneten Katalysatoren.

Die Oxidierbarkeit von Benzol an Vanadium-/Molybdän-/Wolframoxidkatalysatoren mit Distickstoffmonoxid ist schon seit dem Jahre 1982 (Iwamoto) bekannt. Zeolithkatalysatoren vom Typ ZSM-5 wurden für die Direktoxidation von Benzol mit Distickstoffmonoxid im Jahre 1988 (Gubelmann, Fa. Rhone-Poulenc) entdeckt. Die Wirkungsweise von Zeolithen wird grundsätzlich auf ihr mikroporöses Kanalsystem mit Porenabmessungen in der Größe der zu oxidierenden Moleküle zurückgeführt.

Der Einsatz von eisenhaltigen Zeolithkatalysatoren vom Typ ZSM-5 für die Oxidation von Benzol mit Distickstoffmonoxid ist in J. Phys. Chem. 1994, 98, 7548-7550 von Volodin, Bolshov und Panov beschrieben.

Auch in folgenden Unterlagen ist die Oxidation an Zeolithen, insbesondere dem Typ ZSM-5 beschrieben:
3rd World Congress on Oxidation, 1997 Elsevier Science B.V., R.K. Graselli et al. (Editors),
M. Häfele et al. (Universität Erlangen-Nürnberg) und
G.I. Panov et al., Applied Catalysis A: General, 98 (1993) 1-20

Als Verfahrenstechnisch am weitesten entwickelt gilt die Reaktion von Distickstoffmonoxid an sauren Zeolithen vom Typ ZSM-5 und ZSM-11 mit verschiedenen Metallzusätzen (z.B. Eisen). Die Reaktion wird üblicherweise unter Normaldruck bei Temperaturen von 300 - 450 °C durchgeführt.

Nachteilig bei Zeolithen ist, daß aufgrund des vollständig kristallinen Aufbaus die Variation der Porengröße nicht kontinuierlich dem zu oxidierenden Molekül angepaßt werden kann, sondern abhängig vom sich einstellenden Kristalltyp nur stufenweise möglich ist. Das bedeutet, daß sich Zeolithe auf das jeweilige Oxidationsproblem nicht spezifisch anpassen lassen. Auch die Oberflächenpolarität, über deren Einstellung bestimmte Reaktionsmechanismen vorstellbar sind, ist bei Zeolithen kaum variierbar. Die Literatur, wie z.B. US-Patente Nr. 5.110.995 oder Nr. 5.055.623 beschreiben einen Zeolith- Typ (die Pentasile ZSM-5 und ZSM-11) für die Herstellung vieler Phenolderivate. Zwar können Zeolithe bzw. saure Zeolithe mit verschiedenen Metallen modifiziert werden. Dies kann jedoch das geschilderte Problem allenfalls verbessern, aber nicht grundsätzlich lösen. So kann beispielsweise ein Austausch eines Wasserstoffatoms durch ein Natriumatom in einem sauren Zeolithen erfolgen. Im eisenhaltigen Zeolithen wurde hierbei kein Aktivitätsverlust beobachtet, während sich bei einem Ersatz des Eisens durch Aluminium die Aktivität bei einem Austausch eines Wasserstoffatoms durch ein Natriumatom erniedrigt. Insgesamt können Umsatz und Selektivität nach wie vor aber nicht befriedigen.

Die Veränderung anderer Parameter wie höhere Reaktionstemperatur (Zeolithe gelten bis etwa 800 °C als temperaturstabil) führt zwar zu höheren Benzolumsätzen, aber auch zu geringerer Selektivität und stärkerer Desaktivierung des Katalysators. Bei einer Erhöhung der Partialdrucks an N₂O kann auch der Benzolumsatz erhöht werden, jedoch geht dies ebenfalls auf Kosten der Selektivität. Eine graduelle Verbesserung ist allenfalls durch die Erhöhung des Partialdrucks an Benzol erzielbar. Die Selektivität und die erhaltenen Phenolmengen steigen etwas an. Trotzdem ist es nach wie vor wünschenswert, die Selektivität und den Umsatzgrad weiter zu steigern.

Die bei den üblicherweise eingesetzten Temperaturen auftretende relativ rasche Verkokung des Katalysators führt zu einem Aktivitätsverlust und der Katalysator muß deshalb relativ häufig regeneriert werden muß (etwa alle 48 Stunden).

Das in den oben beschriebenen Verfahren zur katalytischen Oxidation von Benzolen eingesetzte Distickstoffmonoxid muß einen sehr hohen Reinheitsgrad aufweisen. Verunreinigungen, wie Sauerstoff oder hydrophile Gase wie Wasserdampf oder Ammoniak können den Zeolithkatalysator bis zur Wirkungslosigkeit inaktivieren. Lediglich inerte Gase wie Edelgase oder Stickstoff sind als Beimengungen tolerabel.

Für Distickstoffmonoxid kommen verschiedene Quellen in Betracht. Die katalytische Zersetzung von Ammoniumnitrat bei 100-160 °C mit Mangan-, Kupfer- Blei-, Wismut-, Kobalt- und Nickelkatalysatoren liefert eine Mischung aus Distickstoffmonoxid, Stickstoffoxid und Stickstoffdioxid, so daß das Gas nicht direkt für die Oxidation von Benzol eingesetzt werden kann.
Etwas günstiger ist die Oxidation von Ammoniak mit Sauerstoff an Platin- oder Wismutoxidkatalysatoren bei 200-500 °C sowie die Umsetzung von Stickstoffoxid mit Kohlenmonoxid an Platinkatalysatoren. Im ersten Fall entsteht jedoch als Nebenprodukt Wasser, im zweiten Fall Kohlendioxid. Das auf diese Weise hergestellte Distickstoffmonoxid kann auch nicht direkt für die Benzoloxidation eingesetzt werden. Ebenso kann das bei der Adipinsäureherstellung anfallende Distickstoffmonoxid fiir die Oxidation nicht direkt verwendet werden, sondern muß einem separaten Reinigungsschritt unterzogen werden. Insbesondere stören der im Abgas enthaltenene Sauerstoff und das NOₓ.

Die Reinheit des Distickstoffmonoxids ist auch vor dem Hintergrund zu sehen, daß Zeolithe Wasser aufnehmen, was ihre katalytische Wirkung reduziert. Einige wenige Zeolithstrukturen lassen sich zwar durch Desaluminierung hydrophobieren, die Auswahl an geigneten Zeolithen wird jedoch hierdurch weiter begrenzt. Die Desaluminierung ist weiterhin ein zusätzlicher Verfahrensschritt und führt zu unerwünschten amorphen Anteilen im Zeolithen. Außerdem läßt sich das Ausmaß der Desaluminierung nicht gezielt einstellen, so daß im Hinblick auf das Verfahren dieses empirisch ermittelt werden muß. Das bedeutet, daß schwankende Oxidationsmittelqualitäten wie z. B. unterschiedliche Beimengungen von Wasserdampf nicht einsetzbar sind.

Aufgabe der vorliegenden Erfindung war es daher, ein wirtschaftliches Verfahren zur katalytischen Oxidation von aromatischen Verbindungen zu den entsprechenden Hydroxyverbindungen mit hohen Selektivitäten auch bei geringer Distickstoffmonoxidqualität bereitzustellen.

Überraschenderweise wurde gefunden, das poröse Gläser die katalytische Oxidation von aromatischen Verbindungen zu entsprechenden Hydroxyverbindungen auch bei Verwendung von verunreinigtem Distickstoffmonoxid hervorragend katalysieren können.
Gegenstand der vorliegenden Erfindung ist daher ein Verfahren zur Herstellung Hydroxylgruppen enthaltender aromatischer Verbindungen durch katalytische Oxidation, wobei die katalytische Oxidation der aromatischen Verbindung in Gegenwart eines porösen Glases (mikroporösen amorphen Mischmetalloxids) mit einem Distickstoffmonoxid enthaltenden Gas bei einer Temperatur von 100-800 °C durchgeführt wird.

Das erfindungsgemäße Verfahren kann durch Verwendung eines entsprechenden porösen Glases auf die jeweiligen Einsatzbedingungen wie z. B. der Qualität des einzusetzenden Distickstoffmonoxids oder die Art der aromatischen Verbindung optimal angepaßt werden.

Dies war nicht zu erwarten, da die Fachliteratur für einen hohen Benzolumsatz bei gleichzeitiger hoher Selektivität zu Phenol "besondere" Zeolithe z.B. vom Pentasil-Typ wie etwa ZSM-5 und ZSM-11 nahe legt.

Diese Zeolithe besitzen eine dreidimensionale Struktur ("Käfigstruktur") und daher eine große Oberfläche. Das Porensystem der im erfindungsgemäßen Verfahren eingesetzten porösen Gläser ist jedoch überwiegend anders strukturiert, so daß sie gegenüber Zeolithen in der Regel keinen dreidimensionalen Zugang erlauben und man daher eine (wenn überhaupt) wesentlich reduzierte katalytische Wirkung erwarten sollte.

Inwiefern das durch das Herstellverfahren bedingte Kanalsystem oder die Zusammensetzung der im vorliegenden Verfahren eingesetzten porösen Gläser die Ursache deren katalytischer Wirkung ist, mag dahingestellt bleiben, das erfindungsgemäße Verfahren führt jedoch auch bei einem Einsatz von Distickstoffmonoxid minderer Qualität zu hohen Umsätzen bei sehr guten Selektivitäten.

Als Distickstoffmonoxid enthaltendes Gas kann eine Mischung aus 5 bis 100 Vol.-% Distickstoffmonoxid und 0 bis 95 Vol.-% eines weiteren Gases eingesetzt werden.

Der Distickstoffmonoxidgehalt des im erfindungsgemäßen Verfahren eingesetzten Gases kann ebenso zwischen 80 und 100 Vol.-%, verbunden mit 0 bis 20 Vol.-% eines weiteren Gases liegen.

Als weitere Gase können Luft, Sauerstoff, Stickstoff, Edelgase, Kohlendioxid, Wasserdampf oder Ammoniak oder deren Gemische zum Einsatz kommen.

Das eingesetzte Distickstoffmonoxid enthaltende Gas kann auch organische oder anorganische Verunreinigungen, z.B. aus einem Oxidationsprozeß (z.B. N₂O-Herstellung in situ oder N₂O-haltiges Abgas aus der Adipinsäureherstellung) enthalten.

In einer besonderen Ausführungsform der vorliegenden Erfindung wird als aromatische Verbindung Benzol verwendet, es ist jedoch auch die Oxidation anderer Aromaten wie Toluole, Xylole oder halogenierte Benzole sowie die Oxidation mehrkerniger Aromaten wie z. B. Naphthalin möglich.

Überraschend wurde auch bei an der Oberfläche unpolar modifizierten porösen Gläsern beobachtet, daß der Benzolumsatz unterhalb 375 °C (z.B. 350 °C) fast genauso so hoch ist wie oberhalb 375 °C. Die Selektivität steigt bei tieferen Temperaturen erwartungsgemäß an. Bei Zeolith-katalysierten System fällt üblicherweise der Benzolumsatz mit sinkender Temperatur stark ab. Unter vergleichbaren Bedingungen verhält sich ein Zeolith also wesentlich ungünstiger.

Das erfindungsgemäße Verfahren wird bevorzugt bei Temperaturen von 200-700 °C, besonders bevorzugt bei 250-550 °C durchgeführt.
Die Herstellung der im erfindungsgemäßen Verfahren eingesetzten porösen Gläsern bzw. mikroporösen amorphen Mischmetalloxiden kann gemäß DE 195 06 843 A 1 erfolgen.

Die im erfindungsgemäßen Verfahren eingesetzten porösen Gläser werden nach dem Sol-Gel-Verfahren hergestellt.

In einer Ausführungsform der vorliegenden Erfindung bestehen die porösen Gläser zu 50 -100 Gewichts-% aus Oxiden von Elementen aus der 3. Hauptgruppe, der 4. Hauptgruppe, der 3. Nebengruppe oder aus der 4. Nebengruppe des Periodensystems, einschließlich der Lanthanoiden und Actinoiden.

In einer anderen Ausführungsform der vorliegenden Erfindung kann die Mischmetalloxidmatrix der porösen Gläser mindestens 50 Gewichts-% Verbindungen der Elemente Titan, Silizium, Vanadium, Aluminium, Zirkon oder Cer und/oder bis zu 50 Gewichts-% einer oder mehrerer Metalloxide in sehr feiner bis atomarer Verteilung aus der Gruppe der Metalle Molybdän, Zinn, Zink, Vanadium, Mangan, Eisen, Kobalt, Nickel, Arsen, Blei, Antimon, Wismut, Ruthenium, Rhenium, Chrom, Wolfram, Niob, Hafnium, Lanthan, Cer, Gadolinium, Gallium, Indium, Thallium Silber, Kupfer, Lithium, Kalium, Natrium, Beryllium, Magnesium, Calcium, Strontium und Barium enthalten.

Bevorzugt enthält die Mischmetalloxidmatrix der porösen Gläser mindestens eine, besonders bevorzugt mindestens zwei der Verbindungen aus der Gruppe SiO₂, TiO₂, Al₂O₃, Vanadiumoxid, Zirkoniumoxid, Ceroxid, Spinell, Mullit, Siliziumcarbid, Siliziumnitrid und Titannitrit.

Weiterhin kann die Mischmetalloxidmatrix zusätzlich bis zu 10 Gewichts-% eines der Metalle Platin, Rhodium, Iridium, Osmium, Silber, Gold, Kupfer, Nickel, Palladium und Kobalt in hochdisperser Form in metallischem oder oxidiertem Zustand enthalten.

Die porösen Gläser sind erhältlich durch saure oder Fluorid-katalysierte lineare Polymerisation oder Polykondensation hydrolisierbarer, löslicher Verbindungen oben genannter Metalle und Oxide. Vorzugsweise werden Alkoxy-, gemischte Alkoxyalkyl-, Alkoxyoxo- oder Acetylacetonat-Derivate der beschriebenen Metalle oder Metalloxide im sauren bis neutralen pH-Bereich im Sol-Gel-Verfahren eingesetzt. Danach erfolgt ein mildes Trocknen und langsames Calzinieren, wobei das Ende der Calzinierungstemperatur bei 120-800 °C liegt.

Eine alternative Herstellungsmethode für diese Art von Verbindungen stellt das Thermolyseverfahren dar (K. W. Terry et al. J. Am. Chem. Soc. 1997, 119, 9745-9756). Nachteilig ist hier aber gegenüber dem Sol-Gel-Verfahren die verminderte Variationsmöglichkeit bei der Herstellung, so daß im Endprodukt nur einige wenige Elementverhältnisse möglich sind.

Die Herstellung unpolarer bzw. hydrophober poröser Gläser geht z. B aus der Patentschrift DE 195 45 042 A 1 hervor. Die Polarität der inneren und äußeren Oberfläche poröser Gläser kann beispielsweise dadurch eingestellt werden, daß Alkyl- oder Aryloxysilane mit nicht hydrolisierbaren Alkyl- oder Arylgruppen R' vom Typ R'- Si(OR)₃ mit den anderen Komponenten des Sol-Gel-Prozesses copolykondensiert werden.

R bzw. R' können gleich oder verschieden sein und sind bevorzugt aliphatische Kohlenwasserstoffreste mit bis zu 6 Kohlenstoffatomen wie Methyl-, Ethyl- oder Isopropylgruppen oder Phenylreste.

Das mit dieser nichthydrolysierbaren Gruppe umgesetzte Metalloxid stammt aus der oben genannten Aufzählung von Metallen. Als Liganden werden für die Ausgangsverbindung, also die lösliche Metallverbindung, vorzugsweise Halogenide, Alkoxide, Oxyalkoxide, Carboxylate, Oxalate, Nitrate, Sulfate, Sulfonate, Acetylacetonate, Glykolate oder Aminoalkoxylate verwendet. Das Basismaterial ist SiO₂, Al₂O₃, TiO₂ oder ZrO₂.

Der Einsatz hydrophobierter poröser Gläser ist insbesondere bei einem hohen Wassergehalt des Distickstoffmonoxid von Vorteil, da so eine gleichbleibende hohe Selektivität gewährleistet ist.

Neben porösen Gläsern können auch Zuschlagstoffe eingesetzt werden. Als solche sind auch Trägersysteme oder Gemische auf SiO₂- oder Al₂O₃-Basis zu verstehen. Die Verwendung einer Kombination aus porösen Gläsern und (Kristallinen) Zeolithen ist ebenfalls, wie in den Beispielen gezeigt, ohne Umsatz- und Selektivitätsverlust möglich.

Wichtig ist die Porengröße und die Gesamtoberfläche der verwendeten Gläser. Der Mittelwert der Porengröße sollte zwichen 0.1 und 1.0 nm, ermittelt nach Horvath und Kawazoe (J. Chem. Eng. Jpn. 16 (1983) 470 ff) liegen.

Die Gesamtoberfläche der porösen Gläser im getrockneten Zustand beträgt bevorzugt mindestens 50 m²/g, besonders bevorzugt 50-5000 m²/g, ganz besonders bevorzugt 75-1500 m²/g, jeweils bestimmt nach der BET-Methode gemäß W. F. Maier et al. Tetrahedron 51 (1995) 3787 ff.

### Beispiele:

### 1. Herstellung poröser Gläser

### 1.1 Zirkondioxid-Siliziumdioxid-Glas

1ml Tetrabutoxyzirkonium, 10 ml Tetraethoxysilan und 8 ml Ethanol werden hintereinander ineinander gelöst und 2 ml 8 n Salzsäure unter Rühren zugegeben. Nach erfolgter Gel-Bildung wird das Material auf 65 °C mit einer Heizrate von 0,5 °C/min aufgeheizt, 3 Stunden bei 65 °C gehalten, mit einer Heizrate von 0,2 °C/min auf 250 °C aufgeheizt und weitere 3 Stunden bei dieser Temperatur calziniert. Das Produkt zeigt eine monomodale Porenverteilung.
BET: 500 m²/g
Porendurchmesser: 0,74 nm

### 1.2 Aluminiumoxid-Zirkondioxid-Siliziumdioxid-Glas

0,45 ml Triisobutylaluminium, 2,01 ml Tetra-n-propoxyzirkonium, 27,5 ml Tetraethoxysilan und 25 ml Ethanol werden hintereinander ineinander gelöst und 4,5 ml 0,4 n Salzsäure unter Rühren während 10 min zugegeben. Dabei steigt die Temperatur bis auf 55 °C. Nach erfolgter Gel-Bildung und langsamer Vortrocknung bei Raumtemperatur wird das Material auf 65 °C mit einer Heizrate von 0,5 °C/min aufgeheizt, 3 Stunden bei 65 °C gehalten, mit einer Heizrate von 0,2 °C/min auf 300 °C aufgeheizt und weitere 3 Stunden bei dieser Temperatur calziniert. Das Produkt zeigt eine monomodale Porenverteilung.
BET: 290 m²/g
Porendurchmesser: 0,69 nm

### 1.3 Zirkondioxid-Siliziumdioxid-Glas

1 ml Tetrabutoxyzirkonium, 10 ml Tetraethoxysilan und 8,4 ml Ethanol werden hintereinander ineinander gelöst und 2 ml 2 n Salzsäure unter Rühren zugegeben. Nach erfolgter Gel-Bildung wird das Material auf 65 °C mit einer Heizrate von 0,5 °C/min aufgeheizt, 3 Stunden bei 65 °C gehalten, mit einer Heizrate von 0,2 °C/min auf 250 °C aufgeheizt und weitere 3 Stunden bei dieser Temperatur calziniert. Das Produkt zeigt eine monomodale Porenverteilung.
BET: 250 m²/g
Porendurchmesser: 0,65 nm

### 1.4 Vanadiumoxid-Siliziumdioxid-Glas

1,2 g Vanadylacetylacetonat, 10 ml Tetraethoxysilan und 8 ml Ethanol werden hintereinander ineinander gelöst und 2 ml 8 n Salzsäure unter Rühren zugegeben. Nach erfolgter Gel-Bildung wird das Material auf 65 °C mit einer Heizrate von 0,5 °C/min aufgeheizt, 3 Stunden bei 65 °C gehalten, mit einer Heizrate von 0,2 °C/min auf 250 °C aufgeheizt und weitere 3 Stunden bei dieser Temperatur calziniert. Das Produkt zeigt eine monomodale Porenverteilung.
BET: 550 m²/g
Porendurchmesser: 0,66 nm

### 1.5 Eisenhaltiges Siliziumdioxid-Glas

1,2 g Eisenacetylacetonat, 10 ml Tetraethoxysilan und 8 ml Ethanol werden hintereinander ineinander gelöst und 2 ml 8 n Salzsäure unter Rühren zugegeben. Nach erfolgter Gel-Bildung wird das Material auf 65 °C mit einer Heizrate von 0,5 °C/min aufgeheizt, 3 Stunden bei 65 °C gehalten, mit einer Heizrate von 0,2 °C/min auf 230 °C aufgeheizt und weitere 5 Stunden bei dieser Temperatur calziniert. Das Produkt zeigt eine monomodale Porenverteilung.
BET: 520 m²/g
Porendurchmesser: 0,62 nm

### 1.6 Titandioxid-Siliziumdioxid-Glas

0,14 ml Tetraethoxytitan, 10 ml Tetraethoxysilan und 8 ml Ethanol werden hintereinander ineinander gelöst und 1,8 ml 8 n Salzsäure unter Rühren zugegeben. Nach erfolgter Gel-Bildung wird das Material auf 65 °C mit einer Heizrate von 0,5 °C/min aufgeheizt, 3 Stunden bei 65 °C gehalten, mit einer Heizrate von 0,2 °C/min auf 250 °C aufgeheizt und weitere 3 Stunden bei dieser Temperatur calziniert. Das Produkt zeigt eine monomodale Porenverteilung.
BET: 480 m²/g
Porendurchmesser: 0,67 nm

### 1.7 Hydrophobiertes Titandioxid-Siliziumdioxid-Methylsiliziumsesquioxid-Glas

0,133 ml Tetraisopropoxytitan, 8 ml Tetraethoxysilan, 1,8 ml Methyltriethoxysilan und 7,9 ml Ethanol werden nacheinander ineinander gelöst und 1,98 ml 8 n Salzsäure unter Rühren zugegeben. Nach Gel-Bildung und erfolgter Erhärtung des Gels wird dieses unter Schutzgas auf 65 °C mit einer Heizrate von 0,2 °C/min aufgeheizt. 3 Stunden bei 65 °C gehalten, mit einer Heizrate von 0,2 °C/min auf 250 °C aufgeheizt und weitere 3 Stunden bei dieser Temperatur calziniert. Das Produkt zeigt eine monomodale Porenverteilung.
BET: 540 m²/g
Porendurchmesser: 0,70 nm

### 1.8 Hydrophobiertes eisenhaltiges Siliziumdioxid-Methylsiliziumsesquioxid-Glas

8 ml Tetraethoxysilan, 1,8 ml Methyltriethoxysilan, 0,5 g Eisenacetylacetonat und 8 ml Ethanol werden nacheinander ineinander gelöst und 2 ml 8 n Salzsäure unter Rühren zugegeben. Nach Gel-Bildung und erfolgter Erhärtung des Gels wird dieses unter Schutzgas auf 65 °C mit einer Heizrate von 0,2 °C/min aufgeheizt. 3 Stunden bei 65 °C gehalten, mit einer Heizrate von 0,2 °C/min auf 230 °C aufgeheizt und weitere 5 Stunden bei dieser Temperatur calziniert. Das Produkt zeigt eine monomodale Porenverteilung.
BET: 420 m²/g
Porendurchmesser: 0,61 nm

### 2. Umsetzung der Katalysatoren mit Benzol und Distickstoffmonoxid/Begleitgas

### 2.1 Versuchsbedingungen

In einen röhrenförmigen Reaktor mit dem Innendurchmesser von 8 mm werden 2 cm³ Katalysator eingefüllt. Der Katalysator wird vorher auf eine Korngröße von 500 -1000 µm vermahlen. Der Reaktionsraum wird auf die vorgegebene Temperatur erwärmt. Die Kontrolle der Temperatur erfolgt über ein Thermoelement. Die Benzol- und die Gasmenge werden kontinuierlich gasförmig zugegeben, wobei der Gasstrom auf 60 cm³/min eingestellt wird. Der Druck liegt im Bereich von 760 Torr. Das Molverhältnis von Benzol zu Oxidationsmittel im Gasraum beträgt hierbei 1:5. Für die Laborversuche wird als Trägergas Stickstoff verwendet. Die Analyse der Gaszusammensetzung erfolgt über ein GC-/MS-System. Als Zeolithe kommen Fe-ZSM-5 der Fa. UOP und ZSM-5 (eisenfrei) der Fa. VAW zum Einsatz.

### 2.2 Ergebnisse

| | Versuchsnummer | Katalysator | Temperatur (°C) | Zusammensetzung Oxidationsmittel (Vol-%) | Benzolumsatz (%) | Selektivität (%) |
|---|---|---|---|---|---|---|
| | | | | | | |
| nicht erfindungsgemäß | 1. | eisenhaltiger Zeolith ZSM-5 | 375 | 100 N₂O | 7 | 97 |
| nicht erfindungsgemäß | 2. | eisenhaltiger Zeolith ZSM-5 | 425 | 100 N₂O | 13 | 85 |
| nicht erfindungsgemäß | 3. | Zeolith ZSM-5 | 375 | 99 N₂O/1 H₂O | 0 | 0 |
| erfindungsgemäß | 4. | 1.1 | 375 | 99 N₂O/1 H₂O | 0,5 | 50 |
| erfindungsgemäß | 5. | 100 % ZSM-5/ 100 % 1.1 | 375 | 99 N₂O/1 H₂O | 2 | 59 |
| erfindungsgemäß | 6. | 1.1 | 375 | 100 N₂O | 2 | 55 |
| erfindungsgemäß | 7. | 1.2 | 375 | 100 N₂O | 2 | 67 |
| erfindungsgemäß | 8 | 1.3 | 375 | 100 N₂O | 1 | 55 |
| erfindungsgemäß | 9. | 1.4 | 375 | 100 N₂O | 2 | 49 |
| erfindungsgemäß | 10. | 1.5 | 375 | 100 N₂O | 11 | 89 |
| erfindungsgemäß | 11. | 1.6 | 375 | 100 N₂O | 1 | 51 |
| erfindungsgemäß | 12. | 1.7 | 375 | 100 N₂O | 6 | 91 |
| erfindungsgemäß | 13. | 1.8 | 375 | 100 N₂O | 13 | 95 |
| erfindungsgemäß | 14. | 1.8 | 350 | 100 N₂O | 12 | 99 |
| erfindungsgemäß | 15. | 1.8 | 375 | 90 N₂O/10 H₂O | 10 | 96 |
| nicht erfindungsgemäß | 16. | eisenhaltiger Zeolith ZSM-5 | 375 | 90 N₂O/10 H₂O | 0 | 0 |
| erfindungsgemäß | 17. | 1.8 | 375 | 99 N₂O/1 NH₃ | 3 | 85 |
| erfindungsgemäß | 18. | 1.8 | 375 | 95 N₂O/5 Luft | 4 | 92 |
| erfindungsgemäß | 19. | 1.8 | 375 | 99 N₂O/1 O₂ | 5 | 79 |

## Patentansprüche

1. Verfahren zur Herstellung Hydroxylgruppen enthaltender aromatischer Verbindungen durch katalytische Oxidation ,
**dadurch gekennzeichnet,**
**daß** die katalytische Oxidation der aromatischen Verbindung in Gegenwart eines porösen Glases (mikroporösen amorphen Mischmetalloxids) mit einem Distickstoffmonoxid enthaltenden Gas bei einer Temperatur von 100-800 °C durchgeführt wird.

2. Verfahren nach Anspruch 1,
**dadurch gekennzeichnet,**
**daß** als aromatische Verbindung Benzol eingesetzt wird.

3. Verfahren nach einem der Ansprüche 1 oder 2,
**dadurch gekennzeichnet,**
**daß** das Distickstoffmonoxid enthaltende Gas 5 bis 100 Vol.-% Distickstoffmonoxid und 0 bis 95 Vol.-% eines weiteren Gases enthält.

4. Verfahren nach einem der Ansprüche 1 oder 2,
**dadurch gekennzeichnet,**
**daß** das Distickstoffmonoxid enthaltende Gas 80 bis 100 Vol.-% Distickstoffmonoxid und 0 bis 20 Vol.-% eines weiteren Gases enthält.

5. Verfahren nach einem der Ansprüche 3 oder 4,
**dadurch gekennzeichnet,**
**daß** das weitere Gas aus Luft, Sauerstoff, Stickstoff, Edelgasen, Kohlendioxid, Wasserdampf oder Ammoniak oder deren Gemischen besteht.

6. Verfahren nach einem der Ansprüche 1 bis 5,
**dadurch gekennzeichnet,**
**daß** der Mittelwert der Porengröße des porösen Glases zwischen 0.1 und 1.0 nm liegt.

7. Verfahren nach einem der Ansprüche 1 bis 6,
**dadurch gekennzeichnet**
**daß** die katalytische Oxidation bei einer Temperatur von 200-700 °C durchgeführt wird.

8. Verfahren nach Anspruch 7,
**dadurch gekennzeichnet,**
**daß** die katalytische Oxidation bei einer Temperatur von 250-550 °C durchgeführt wird.

9. Verfahren nach einem der Ansprüche 1 bis 8,
**dadurch gekennzeichnet,**
**daß** die Gesamtoberfläche des porösen Glases im trockenen Zustand mindestens 50 m²/g beträgt.

10. Verfahren nach Anspruch 9,
**dadurch gekennzeichnet,**
**daß** die Gesamtoberfläche des porösen Glases im trockenen Zustand 50-5000 m²/g beträgt.

11. Verfahren nach Anspruch 9,
**dadurch gekennzeichnet,**
**daß** die Gesamtoberfläche des porösen Glases im trockenen Zustand 75-1500 m²/g beträgt.

12. Verfahren nach einem der Ansprüche 1 bis 11,
**dadurch gekennzeichnet,**
**daß** das poröse Glas nach dem Sol-Gel-Verfahren hergestellt wird und zu mindestens 50 Gew.-% aus Oxiden von Elementen aus der 3. Hauptgruppe, der 4. Hauptgruppe, der 3. Nebengruppe oder der 4. Nebengruppe des Periodensystems, einschließlich der Lanthanoiden und Actinoiden, besteht.

## Claims

1. A process for preparing hydroxyl-containing aromatic compounds by catalytic oxidation, **characterized in that** the catalytic oxidation of the aromatic compound is carried out with a dinitrogen-monoxide-containing gas in the presence of a porous glass at a temperature of 100-800°C.

2. A process according to claim 1, **characterized in that** the aromatic compound used is benzene.

3. A process according to claim 1 or 2, **characterized in that** the dinitrogen-monoxide-containing gas comprises from 5 to 100% by volume of dinitrogen monoxide and from 0 to 95% by volume of another gas.

4. A process according to claim 1 or 2, **characterized in that** the dinitrogen-monoxide-containing gas comprises from 80 to 100% by volume of dinitrogen monoxide and from 0 to 20% by volume of another gas.

5. A process according to claim 3 or 4, **characterized in that** the other gas consists of air, oxygen, nitrogen, noble gases, carbon dioxide, steam or ammonia or mixtures thereof.

6. A process according to any one of claims 1 to 5, **characterized in that** the mean pore size of the porous glass is from 0.1 to 1.0 nm.

7. A process according to any one of claims 1 to 6, **characterized in that** the catalytic oxidation is carried out at a temperature of 200-700°C.

8. A process according to claim 7, **characterized in that** the catalytic oxidation is carried out at a temperature of 250-550°C.

9. A process according to any one of claims 1 to 8, **characterized in that** the total surface area of the porous glass in the dry state is at least 50 m²/g.

10. A process according to claim 9, **characterized in that** the total surface area of the porous glass in the dry state is 50-5000 m²/g.

11. A process according to claim 9, **characterized in that** the total surface area of the porous glass in the dry state is 75-1500 m²/g.

12. A process according to any one of claims 1 to 11, **characterized in that** the porous glass is prepared by the sol-gel process and consists of at least 50% by weight of oxides of elements of main group 3, of main group 4, of subgroup 3 or of subgroup 4 of the Periodic Table of the Elements, inclusive of the lanthanides and actinides.

## Revendications

1. Procédé de préparation de composés aromatiques contenant des groupes hydroxyle, par oxydation catalytique,
**caractérisé en ce qu'**
on effectue l'oxydation catalytique du composé aromatique en présence d'un verre poreux d'oxyde métallique mixte amorphe microporeux avec un gaz contenant du monoxyde de diazote à une température allant de 100 à 800°C.

2. Procédé selon la revendication 1,
**caractérisé en ce qu'**
on met en oeuvre comme composé aromatique, du benzène.

3. Procédé selon la revendication 1 ou la revendication 2,
**caractérisé en ce que**
le gaz contenant du monoxyde de diazote renferme de 5 à 100 % en volume de monoxyde de diazote et de 0 à 95 % en volume d'un autre gaz.

4. Procédé selon l'une des revendications 1 ou 2,
**caractérisé en ce que**
le gaz contenant du monoxyde de diazote renferme de 80 à 100 % en volume de monoxyde de diazote et de 0 à 20 % en volume d'un autre gaz.

5. Procédé selon l'une des revendications 3 ou 4,
**caractérisé en ce que**
l'autre gaz consiste en de l'air, de l'oxygène, de l'azote, des gaz nobles, du dioxyde de carbone, de la vapeur d'eau ou de l'ammoniac ou en leurs mélanges.

6. Procédé selon l'une quelconque des revendications 1 à 5,
**caractérisé en ce que**
la valeur moyenne de la taille des pores du verre poreux se situe entre 0,1 à 1 nm.

7. Procédé selon l'une quelconque des revendications 1 à 6,
**caractérisé en ce qu'**
on effectue l'oxydation catalytique à une température de 200 à 700°C.

8. Procédé selon la revendication 7,
**caractérisé en ce qu'**
on effectue l'oxydation catalytique à une température de 250 à 550°C.

9. Procédé selon l'une quelconque des revendications 1 à 8,
**caractérisé en ce que**
la surface totale du verre poreux à l'état séché est au moins à 50 m²/g.

10. Procédé selon la revendication 9,
**caractérisé en ce que**
la surface totale du verre poreux à l'état séché est de 50 - 5000 m²/g.

11. Procédé selon la revendication 9,
**caractérisé en ce que**
la surface totale du verre poreux à l'état séché, est de 75 - 1500 m²/g.

12. Procédé selon l'une quelconque des revendications 1 à 11,
**caractérisé en ce que**
le verre poreux est produit selon le procédé sol-gel et consiste pour au moins 50 % en des oxydes des éléments du 3ème groupe principal, du 4^{ème} groupe principal, du 3ème sous groupe ou du 4ème sous groupe du système périodique, y compris des lanthanides et des actinides.
